(19) Europäisches Patentamt
European Patent Office
Office européen des brevets

(11) Veröffentlichungsnummer: **0 057 280**
**B1**

(12) # EUROPÄISCHE PATENTSCHRIFT

(45) Veröffentlichungstag der Patentschrift: **13.11.85**

(51) Int. Cl.⁴: **C 07 D 239/52**

(21) Anmeldenummer: **81110395.1**

(22) Anmeldetag: **12.12.81**

(54) **Verfahren zur Herstellung von 4-Hydroxy-2-alkoxypyrimidinen.**

(30) Priorität: **31.01.81 DE 3103348**

(43) Veröffentlichungstag der Anmeldung: **11.08.82 Patentblatt 82/32**

(45) Bekanntmachung des Hinweises auf die Patenterteilung: **13.11.85 Patentblatt 85/46**

(84) Benannte Vertragsstaaten: **AT BE CH DE FR GB IT LI NL SE**

(56) Entgegenhaltungen:
**DE - A - 2 008 874**
**DE - A - 2 060 875**

**J. ORG. CHEM., Bd. 35, Nr. 11, 1970, J.L. WONG und D.S. FUCHS "Competitive Isomerization and Dealkylation of 2,4-Dialkoxypyrimidines in Aqueous and Nonaqueous Media" Seiten 3786-3791**

**Die Akte enthält technische Angaben, die nach dem Eingang der Anmeldung eingereicht wurden und die nicht in dieser Patentschrift enthalten sind.**

(73) Patentinhaber: **DYNAMIT NOBEL AKTIENGESELLSCHAFT, Postfach 1209, D-5210 Troisdorf, Bez. Köln (DE)**

(72) Erfinder: **Peeters, Hermann, Dr., Porzer Strasse 1, D-5216 Niederkassel 3 (DE)**

EP 0 057 280 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von 4-Hydroxy-2-alkoxypyrimidinen.

4-Hydroxy-2-alkoxypyrimidine der Formel (1) sind bislang nur nach einem sehr aufwendigen Verfahren in schlechter Ausbeute zugänglich durch Umsetzung von Uracil zu 2,4-Dichlorpyrimidin, Substitution mit Alkoholat zu 2,4-Dialkoxypyrimidin und anschließende selektive Verseifung (J. org. Chem. 35, (1970) 3786).

5-Fluor-4-hydroxy-2-alkoxypyrimidine entstehen auch aus Säureadditionssalzen des o-Alkylisoharnstoffs und Natrium-3-hydroxy-2-fluoracrylsäureestern nach der US-PS 2 802 005, jedoch muß diese Umsetzung unter wasserfreien Bedingungen ablaufen.

Der vorliegenden Erfindung lag die Aufgabe zu Grunde, ein einfaches und kostengünstiges Verfahren zur Herstellung von 4-Hydroxy-2-alkoxypyrimidinen der Formel (1) zu schaffen.

Aus der DE-AS-2 008 874 war die Herstellung von Pyrimidin-Derivaten aus Ketoestern und Harnstoff bekannt, jedoch konnte daraus nicht hergeleitet werden, wie die andersartigen Pyrimidin-Derivate nach dem vorliegenden Patentanspruch aus andersartigen Ausgangsstoffen hergestellt werden können.

Gegenstand der Erfindung ist ein Verfahren zur Herstellung von substituierten und unsubstituierten 4-Hydroxi-2-alkoxypyrimidinen der Formel

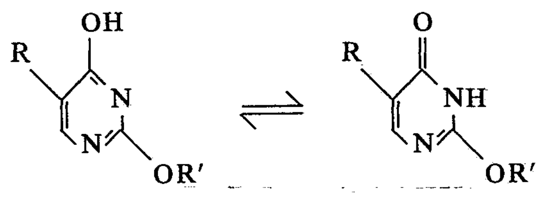

(1)

worin

R ein Wasserstoffatom, geradkettige, verzweigte oder cyclische Alkylreste mit 1 bis 20 Kohlenstoffatomen, geradkettige oder verzweigte Reste $(CH_2)_nCN$, $(CH_2)_nCOOR''$, $(CH_2)_n{-}NH_2$, $(CH_2)_nOR''$, $(CH_2)_n{-}Cyc$, wobei Cyc ein isocyclischer oder heterocyclischer Ring mit ein- oder mehrcyclischer Struktur oder ein aromatischer oder heteroaromatischer Ring mit ein oder mehrcyclischer Struktur, der gegebenenfalls Substituenten am Ring tragen kann,

R″ Alkylreste mit 1 bis 12 Kohlenstoffatomen oder Reste einwertiger Phenole und $n = 1$ bis 5 ist, und

R′ geradkettige, verzweigte oder cyclische Alkylreste mit 1 bis 10 Kohlenstoffatomen oder $(CH_2)_nCyc$, wobei n und Cyc die oben angegebene Bedeutung haben, bedeutet, welches dadurch gekennzeichnet ist, daß eine Verbindung der Formel

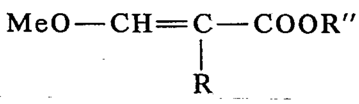

(2)

worin R und R″ dieselbe Bedeutung wie in der Formel (1) haben und Me ein Alkalimetall ist, mit einem Isoharnstoff der Formel

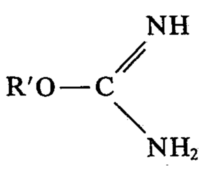

(3)

worin R′ dieselbe Bedeutung wie in der Formel (1) hat, bzw. mit einem Säureadditionssalz des Isoharnstoffs der Formel

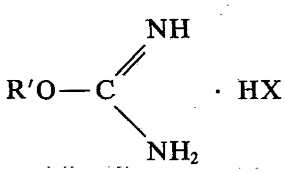

(4)

worin R′ dieselbe Bedeutung wie in der Formel (1) hat und HX eine ein- oder mehrbasische anorganische oder organische Säure bedeutet, umgesetzt wird.

Alkali-3-hydroxyacrylsäureester der Formel 2 sind entsprechend der DE-PS 2 952 070 zugängig, bevorzugt von diesen sind solche, in denen die Gruppe R Wasserstoff, niedere Alkylreste von 1 bis 3 C-Atomen, ein Benzylrest oder ein niederer Alkoxyalkylrest mit 1 bis 3 C-Atomen im Alkyl- und

Alkoxyrest sein kann, besonders der Methoxymethylrest, bedeuten. Je nach Verwendungszweck der substituierten Pyrimidine kann jedoch ein weiterer Rest R in 5-Stellung erwünscht und bevorzugt sein.

Als Alkoholrest R'' der Ester kommen bevorzugt der Methyl- und Ethylrest in Frage.

Bevorzugte Alkylisoharnstoffe sind die Methyl- und Ethylisoharnstoffe, jedoch können je nach Verwendungszweck der Produkte auch weitere Alkylreste oder cyclische Reste bevorzugt sein.

Der o-Alkylisoharnstoff wird bevorzugt als Säureadditionssalz, beispielsweise als Sulfat, Hydrogensulfat oder Hydrochlorid eingesetzt.

Sehr bevorzugt wird die Reaktion zur Erhöhung der Ausbeute, besonders bei Verwendung der Säureadditionssalze des o-Alkylisoharnstoffs in Gegenwart einer anorganischen oder organischen Base durchgeführt. Die Menge der eingesetzten Base beträgt 0,1 bis 3, vorzugsweise 0,5 bis 2 Äquivalente pro Mol o-Alkylisoharnstoff.

Bevorzugt wird, bezogen auf die Säurekomponente der Säureadditionssalze, die äquivalente Menge Base bzw. ein geringer Überschuß der Base bis zu 30% zugesetzt werden.

Als Basen sind sehr bevorzugt die Alkalihydroxide, besonders Natriumhydroxid oder ggf. Kaliumhydroxid, ggf. die Erdalkalihydroxide, wie Ca oder Mg-hydroxide, die Alkalialkoholate mit 1 bis 4 C-Atomen im Alkoholatrest, besonders die Methylate und Ethylate sowie ggf. die entsprechenden Erdalkalialkoholate; andere Basen, wie die organischen Basen Pyridin, Pyridin- oder Pyrimidinabkömmlinge sind möglich aber wenig zweckmäßig.

Sehr bevorzugt ist weiterhin die Verwendung von Wasser als Lösungsmittel oder Mischungen von Wasser und organischen, bevorzugt polaren Lösungsmitteln, besonders Alkoholen mit 1 bis 3 C-Atomen.

In Gegenwart von Wasser wird eine sehr einfache Reaktionsführung in homogener Lösung möglich. Sehr zweckmäßig sind besonders wäßrige Lösungen der Alkalihydroxide wie Natronlauge, jedoch können auch wäßrig alkoholische Alkalilaugen verwendet werden. Soweit kein Wasser anwesend ist, sind Lösungen der Alkalihydroxide in Alkoholen oder der Alkalialkoholate, besonders Natriummethylat oder -ethylat, in den entsprechenden Alkoholen zweckmäßig.

Gegebenenfalls können auch andere polare Lösungsmittel in Gegenwart oder Abwesenheit von Basen Verwendung finden.

Das Molverhältnis von Alkali-3-hydroxyarylsäureester zu o-Alkylisoharnstoff — in freier Form oder gebunden als Salz — sollte 3 zu 1 bis 1 zu 3, vorzugsweise 1,5 zu 1 bis 1 zu 1,5, betragen.

Die Reihenfolge der Zugabe der Reaktanden ist beliebig.

Die Konzentration der Reaktionsteilnehmer wird im wesentlichen durch die Löslichkeit der Reaktanden bzw. die Rührbarkeit der Mischung bestimmt, im allgemeinen beträgt die Konzentration 8 bis 0,25 mol/l, vorzugsweise 4 bis 0,5 mol/l.

Die Umsetzung erfolgt im Temperaturbereich von 0°C bis 150°C, vorzugsweise von 10°C bis 100°C. Im allgemeinen wird bei Normaldruck gearbeitet, jedoch sind auch Überdrücke bis 5 bar ggf. möglich.

Die Reaktion ist im allgemeinen innerhalb von 0,25 bis 10 Stunden abgeschlossen.

Das zunächst anfallende Alkalisalz der 4-Hydroxy-2-alkoxypyrimidine kann durch Zugabe von organischen Säuren wie vorzugsweise Essigsäure oder Mineralsäure wie Salzsäure oder Schwefelsäure und Einstellung auf einen pH-Wert von 3 bis 8 in die freie Verbindung überführt werden. Die Aufarbeitung des Ansatzes erfolgt im allgemeinen so, daß die Reaktionslösung angesäuert, das evtl. ausfallende Reaktionsprodukt abgetrennt und die noch Produkt enthaltende Mutterlauge ggf. nach Abziehen des Lösungsmittels extrahiert wird, oder daß nach dem Ansäuern direkt oder nach Abziehen des Lösungsmittels extrahiert wird. Geeignete Extraktionsmittel sind u. a. Butanol, Methyläthylketon, Methylisopropylketon, Chloroform etc.

2-Alkoxy-4-hydroxypyrimidine können als Ausgangsprodukte u. a. für Pharmazeutika dienen. Durch säurekatalysierte Verseifung kann Uracil erhalten werden, das eine breite Anwendung z. B. im medizinischen Bereich oder als Agrochemikalien oder in der Fotografie besitzt (J. org. Chem. 35 (1970) 3786).

Die folgenden Beispiele dienen der weiteren Erläuterung der Erfindung.

## Beispiel 1

30,6 g (0,2 mol) Natrium-3-hydroxyacrylsäureethylester (Gehalt 90%), 22,2 g (0,09 mol) o-Methylisoharnstoffsulfat und 8 g (0,2 mol) Natriumhydroxid werden in 100 ml Wasser 2 Stunden bei Raumtemperatur gerührt. Durch Zugabe von HCl wird auf einen pH-Wert von 5 bis 6 angesäuert und der gebildete Feststoff abgesaugt und getrocknet. Die Mutterlauge wird mit Methylethylketon extrahiert, die organische Phase getrocknet und das Lösungsmittel abdestilliert. Feststoff und Extraktrückstand ergeben zusammen 18,6 g (82,0% d. Th.) 4-Hydroxy-2-methoxypyrimidin.
Charakterisierung durch $^{1}H$-NMR.

## Beispiel 2

30,6 g (0,2 mol) Natrium-3-hydroxyacrylsäureethylester (Gehalt 90%), 31,0 g (0,18 mol) o-Methylisoharnstoffhydrogensulfat und 16 g (0,4 mol) Natriumhydroxid werden in 100 ml Wasser 2 Stunden bei Raumtemperatur gerührt. Nach Aufarbeitung wie in Beispiel 1 werden 19,2 g (84,7% d. Th.) 4-Hydroxy-2-methoxypyrimidin erhalten.

Beispiel 3

30,6 g (0,2 mol) Natrium-3-hydroxyacrylsäureethylester (Gehalt 90%), 31,6 g (0,17 mol) o-Ethylisoharnstoffhydrogensulfat und 16 g (0,4 mol) Natriumhydroxid werden in 100 ml Wasser gelöst und 3 Stunden bei Raumtemperatur gerührt. Nach Aufarbeitung wie im Beispiel 1 werden 17,4 g (73,1% d. Th.) 4-Hydroxy-2-ethoxypyrimidin erhalten.
Charakterisierung durch $^1$H-NMR.

Beispiel 4

26,8 g (0,15 mol) Natrium-3-hydroxy-2-methylacrylsäureethylester (Gehalt 85%), 18,5 g (0,075 mol) o-Methylisoharnstoffsulfat und 6 g (0,15 mol) Natriumhydroxid werden in 50 ml Wasser 2 Stunden auf 60° C erwärmt. Nach dem Abkühlen wird mit Essigsäure ein pH-Wert von 6 eingestellt und mit Chloroform extrahiert. Nach dem Trocknen und Abziehen des Lösungsmittels werden 12,8 g (61,0% d. Th.) 4-Hydroxy-2-methoxy-5-methylpyrimidin erhalten.
Charakterisierung durch $^1$H-NMR.

Beispiel 5

14,3 g (0,05 mol) Natrium-3-hydroxy-2-benzylacrylsäureäthylester (Gehalt 80%), 8,6 g (0,05 mol) o-Methylisoharnstoffhydrogensulfat und 2 g Natriumhydroxid werden in 30 ml Wasser 3 Stunden bei Raumtemperatur gerührt. Nach Reaktionsende wird vom Ungelösten abfiltriert und die Mutterlauge wie im Beispiel 1 aufgearbeitet. Es werden 5,1 g (47,2% d. Th.) 4-Hydroxy-2-methoxy-5-benzyl-pyrimidin erhalten.
Molekülmasse (massenspektrosmetrisch): 216

Beispiel 6

8,0 g (0,035 mol) Natrium-3-hydroxy-2-methoxymethylacrylsäureethylester (Gehalt 80%), 5,6 g (0,021 mol) o-Methylisoharnstoffsulfat und 1,7 g (0,042 mol) Natriumhydroxid werden in 25 ml Wasser 3 Stunden bei 50° C gerührt. Nach dem Abkühlen wird vom Ungelösten abfiltriert und die Mutterlauge mit Salzsäure auf einen pH-Wert von 6 eingestellt. Nach dem Abfiltrieren und Trocknen des Feststoffs erhält man 2,5 g (41,8% d. Th.) 4-Hydroxy-2-methoxy-5-methoxymethylpyrimidin.
Molekülmasse (massenspektroskopisch): 171

Beispiel 7

30,6 g (0,2 mol) Natrium-3-hydroxyacrylsäureethylester (Gehalt 90%), 18,5 g (0,075 mol) o-Methylisoharnstoffsulfat und 8,1 g (0,15 mol) Natriummethylat werden in 200 ml Methanol 2 Stunden zum Rückfluß erhitzt. Nach Reaktionsende wird das Methanol abdestilliert, der Rückstand in Wasser aufgenommen, mit Essigsäure auf den pH-Wert 6 angesäuert und mit Chloroform extrahiert. Nach dem Trocknen der organischen Phase und Abziehen des Lösungsmittels werden 13,3 g (70,4% d. Th.) 4-Hydroxy-2-methoxypyrimidin erhalten.

**Patentansprüche**

1. Verfahren zur Herstellung von substituierten und unsubstituierten 4-Hydroxi-2-alkoxypyrimidinen der Formel

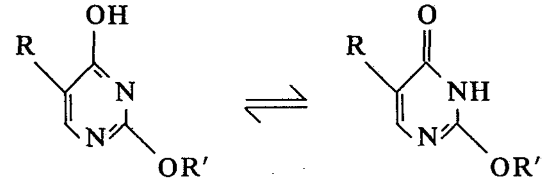

(1)

worin
R ein Wasserstoffatom, geradkettige, verzweigte oder cyclische Alkylreste mit 1 bis 20 Kohlenstoffatomen, geradkettige oder verzweigte Reste $(CH_2)_nCN$, $(CH_2)_nCOOR''$, $(CH_2)_n-NH_2$, $(CH_2)_nOR''$, $(CH_2)_n-Cyc$, wobei Cyc ein isocyclischer oder heterocyclischer Ring mit ein- oder mehrcyclischer Struktur oder ein aromatischer oder heteroaromatischer Ring mit ein oder mehrcyclischer Struktur, der gegebenenfalls Substituenten am Ring tragen kann,
R″ Alkylreste mit 1 bis 12 Kohlenstoffatomen oder Reste einwertiger Phenole und n = 1 bis 5 ist, und
R′ geradkettige, verzweigte oder cyclische Alkylreste mit 1 bis 10 Kohlenstoffatomen oder $(CH_2)_nCyc$, wobei n und Cyc die oben angegebene Bedeutung haben, bedeutet, dadurch gekennzeichnet, daß eine Verbindung der Formel

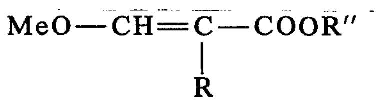
(2)

worin R und R″ dieselbe Bedeutung wie in der Formel (1) haben und Me ein Alkalimetall ist, mit einem Isoharnstoff der Formel

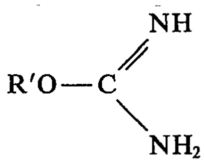
(3)

worin R′ dieselbe Bedeutung wie in der Formel (1) hat, bzw. mit einem Säureadditionssalz des Isoharnstoffs der Formel

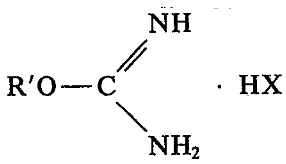
(4)

worin R′ dieselbe Bedeutung wie in der Formel (1) hat und HX eine ein- oder mehrbasische anorganische oder organische Säure bedeutet, umgesetzt wird.

2. Verfahren nach Anspruch 1, dadurch gekennzeichnet, daß die Reaktion in Gegenwart einer Base durchgeführt wird.

3. Verfahren nach einem der Ansprüche 1 oder 2, dadurch gekennzeichnet, daß die Reaktion in Wasser oder einem Gemisch von Wasser und polaren Lösungsmitteln erfolgt.

4. Verfahren nach einem der Ansprüche 1 oder 2, dadurch gekennzeichnet, daß die Reaktion in organischen, bevorzugt polaren Lösungsmitteln erfolgt.

5. Verfahren nach mindestens einem der Ansprüche 1 bis 4, dadurch gekennzeichnet, daß das Molverhältnis von Alkali-3-hydroxy-acrylsäureester zu Isoharnstoff 3 zu 1 bis 1 zu 3, vorzugsweise 1,5 zu 1 bis 1 zu 1,5, beträgt.

6. Verfahren nach den Ansprüchen 1 bis 5, dadurch gekennzeichnet, daß die Reaktionstemperatur 0 bis 150° C, vorzugsweise 10 bis 100° C, beträgt.

**Claims**

1. Process for the production of substituted and unsubstituted 4-hydroxy-2-alkoxypyrimidines of the formula

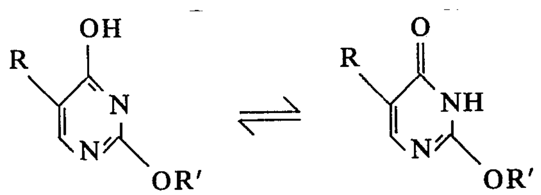
(1)

wherein R is a hydrogen atom, straight-chained, branched or cyclic alkyl residues with 1 to 20 carbon atoms, straight-chained or branched residues $(CH_2)_nCN$, $(CH_2)_nCOOR''$, $(CH_2)_n-NH_2$, $(CH_2)_nOR''$, $(CH_2)_n-Cyc$, wherein Cyc is an isocyclic or heterocyclic ring with mono- or polycyclic structure or an aromatic or heteroaromatic ring with mono- or polycyclic structure, which optionally can carry ring substituents, R″ is alkyl residues with 1 to 12 carbon atoms or residues of monovalent phenols and n = 1

to 5, and R′ denotes straight-chained, branched or cyclic alkyl residues with 1 to 10 carbon atoms or $(CH_2)_nCyc$, wherein n and Cyc have the above-indicated meaning, characterised in that a compound of the formula

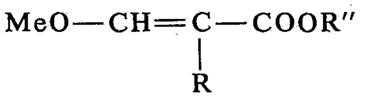

(2)

wherein R and R″ have the same meaning as in formula (1) and Me is an alkali metal, is reacted with an isourea of the formula

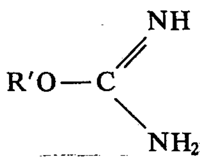

(3)

wherein R′ has the same meaning as in formula (1), or with an acid addition salt of the isourea which is of the formula

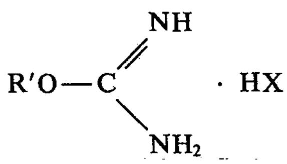

(4)

wherein R′ has the same meaning as in formula (1) and HX denotes a mono- or polybasic inorganic or organic acid.

2. Process according to Claim 1, characterised in that the reaction is carried out in the presence of a base.

3. Process according to one of claims 1 or 2, characterised in that the reaction takes place in water or a mixture of water and polar solvents.

4. Process according to one of claims 1 or 2, characterised in that the reaction takes place in organic, preferably polar solvents.

5. Process according to at least one of claims 1 to 4, characterised in that the molar ratio of alkali-3-hydroxy-acrylic acid ester to isourea amounts to 3 to 1 to 1 to 3, preferably 1.5 to 1 to 1 to 1.5.

6. Process according to claims 1 to 5, characterised in that the reaction temperature amounts to 0 to 150° C, preferably 10 to 100° C.

**Revendications**

1. Procédé de préparation de 4-hydroxy-2-alcoxy-pyrimidines substituées et non substituées de formule

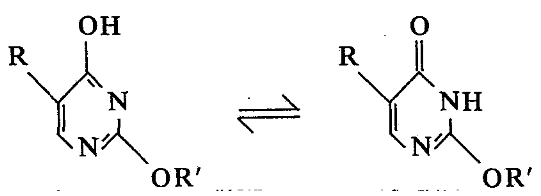

(1)

où R représente un atome d'hydrogène, des radicaux alkyle linéaires, ramifiés ou cycliques à 1—20 atomes de carbone, des radicaux linéaires ou ramifiés $(CH_2)_nCN$, $(CH_2)_nCOOR''$, $(CH_2)_n—NH_2$, $(CH_2)_nOR''$, $(CH_2)_n—Cyc$, Cyc étant un cycle isocyclique ou hétérocyclique à structure mono- ou polycyclique ou un cycle aromatique ou hétéroaromatique à structure mono- ou polycyclique, qui peut porter éventuellement des substituants sur le cycle, R″ représentant des radicaux alkyle à 1—12 atomes de carbone ou des radicaux de monophénols et n étant un nombre de 1 à 5 et R′ représente des radicaux alkyle linéaires, ramifiés ou cycliques à 1—10 atomes de carbone ou des radicaux $(CH_2)_nCyc$, n et Cyc ayant la signification indiquée ci-dessous, procédé caractérisé en ce que l'on fait réagir un composé de formule

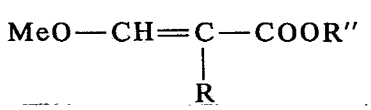

(2)

dans laquelle R et R″ ont la même signification que dans la formule (1) et Me est un métal alcalin, avec une isourée de formule

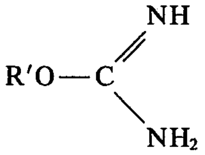

(3)

dans laquelle R′ a la même signification que dans la formule (1) ou avec un sel d'addition d'acide de l'iso-urée de formule

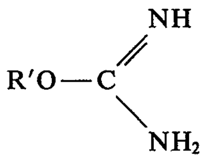

· HX (4)

dans laquelle R′ a la même signification que dans la formule (1) et HX représente un mono- ou polyacide non organique ou organique.

2. Procédé selon la revendication 1, caractérisé en ce que la réaction se fait en présence d'une base.

3. Procédé selon la revendication 1 ou 2, caractérisé en ce que la réaction a lieu dans l'eau ou dans un mélange d'eau et de solvants polaires.

4. Procédé selon la revendication 1 ou 2, caractérisé en ce que la réaction a lieu dans des solvants organiques, de préférence polaires.

5. Procédé selon au moins l'une des revendications 1 à 4, caractérisé en ce que le rapport molaire du dérivé de métal alcalin de l'ester de l'acide 3-hydroxy-acrylique à l'urée est compris entre 3/1 et 1/3, de préférence entre 1,5/1 et 1/1,5.

6. Procédé selon les revendications 1 à 5, caractérisé en ce que la température de réaction est comprise entre 0 et 150° C, de préférence entre 10 et 100° C.